# FASCICULE DE BREVET EUROPEEN

(11) **EP 0 701 549 B1**
(45) Date de publication et mention de la délivrance du brevet: **16.06.1999**
(21) Numéro de dépôt: 94916292.9
(22) Date de dépôt: 17.05.1994
(51) Int. Cl.: C07C 233/47, C07C 231/02, A61K 7/48, A61K 7/06

(54) **PROCEDE DE PREPARATION DE MELANGES D'ACIDES AMINES N-ACYLES**
VERFAHREN ZUR HERSTELLUNG VON MISCHUNGEN VON N-ACYLIERTEN AMINOSAEUREN
METHOD FOR THE PREPARATION OF MIXTURES OF N-ACYLATED AMINOACIDS

(30) Priorité: 17.05.1993 FR 9305906
(43) Date de publication de la demande: 20.03.1996
(73) Titulaire: GIVAUDAN-LAVIROTTE, F-69356 Lyon Cédex 08 (FR)
(72) Inventeur: BERGER, Christian, F-69130 Ecully (FR); GACON, Paul, F-69160 Tassin (FR)
(74) Mandataire: Le Moenner, Gabriel
(86) Numéro de dépôt international: FR9400582
(87) Numéro de publication internationale: WO9426694

(56) Documents cités:
- EP-A- 0 308 278
- WO-A-92/21318
- FR-A- 1 518 665
- FR-A- 2 035 751
- FR-A- 2 254 554
- FR-A- 2 503 144

## Description

La présente invention concerne un procédé pour la préparation simplifiée de mélanges d'acides aminés N-acylés, des mélanges d'acides aminés N-acylés de haute pureté présentant des caractéristiques organoleptiques améliorées, ainsi que des utilisations de ces mélanges.

La préparation de mélanges d'acides aminés N-acylés peut classiquement être réalisée à partir de protéines que l'on hydrolyse pour obtenir un mélange d'acides aminés. Ceux-ci sont alors N-acylés au moyen d'agents d'acylation, puis, le cas échéant, les fonctions carboxyliques des acides aminés N-acylés sont salifiées. De tels procédés sont par exemple décrits dans la demande de brevet WO 92/21318.

L'hydrolyse de ces protéines peut être effectuée au moyen d'un acide fort, tel l'acide chlorhydrique, qu'il convient ultérieurement de neutraliser par une base. Cependant, à l'échelle industrielle, l'hydrolyse est réalisée dans des conditions telles que les acides a aminés constitutifs de la protéine traitée ne sont pas tous obtenus sous forme individualisée. Dans le mélange résultant de l'hydrolyse, on récupère effectivement une quantité importante de condensats d'acides aminés sous forme d'oligopeptides, voire de peptides. Le degré moyen de condensation des acides aminés N-acylés dans un tel mélange peut être compris entre 1,3 et 2, en considérant qu'un degré de condensation de 1 correspond à un acide aminé N-acylé sous forme individualisée.

Pour réaliser une hydrolyse totale de la protéine traitée, des quantités importantes d'acides forts sont requises, induisant alors une augmentation prohibitive du coût global du procédé, ainsi que des problèmes liés à l'environnement. De plus, une hydrolyse totale, en raison des conditions très acides nécessaires à sa mise en oeuvre, peut conduire, d'une part, à la dégradation de certains acides aminés constitutifs de la protéine, tels la tyrosine, l'histidine, le tryptophane ou la sérine, et, d'autre part, à la conversion d'acides aminés N-acylés en certains de leurs dérivés ; ce qui est par exemple le cas de l'asparagine pouvant être transformée en acide aspartique, ou de la glutamine, pouvant être transformée en acide glutamique.

Il apparaît ainsi que les procédés d'hydrolyse classiques ne conduisent pas, soit à un mélange d'acides aminés N-acylés sous forme individualisée substantiellement exempt de condensats d'acides aminés N-acylés, soit à un mélange d'acides aminés N-acylés ne contenant pas tous ou, tout au moins, pas tous les principaux acides aminés N-acylés d'un point de vue quantitatif et/ou qualitatif, qui sont constitutifs de la protéine utilisée comme produit de départ.

Par ailleurs, de nombreuses protéines ne sont présentes dans les organismes qui les contiennent qu'en de faibles teneurs.

Il convient donc d'utiliser une grande quantité de ces organismes pour n'en extraire que de petites quantités de protéines, ceci, en mettant en oeuvre des procédés souvent complexes et coûteux.

On comprend alors que ce type de protéines, chères et difficilement disponibles, ne sont pas utilisées de manière industrielle, en vue de la préparation de mélanges d'acides aminés N-acylés.

C'est d'ailleurs la raison pour laquelle, jusqu'à présent, seuls ont été commercialisés des mélanges d'acides aminés N-acylés, dont les acides aminés sont issus d'hydrolysats de protéines facilement disponibles et de bas prix de revient, telles la caséine, la kératine, le collagène, les protéines de soja ou de blé.

De nombreuses autres sources de protéines n'ont de ce fait jamais pu être mises en oeuvre industriellement pour la préparation de mélanges d'acides aminés N-acylés, alors que de tels mélanges présentent ou sont susceptibles de présenter des intérêts certains dans de nombreux domaines d'applications.

De plus, il a été constaté que les mélanges d'acides aminés N-acylés préparés à partir d'hydrolysats de certaines protéines pouvaient présenter des caractéristiques organoleptiques les rendant peu aptes à leur utilisation dans certains domaines, tels la cosmétique et l'hygiène. En effet, de tels mélanges peuvent présenter une odeur et une coloration parfois très prononcées. Or dans ces domaines, il est requis d'utiliser des composés ne présentant pas d'odeurs désagréables, et, de plus en plus fréquemment, très peu colorés, voire incolores.

Un premier objet de l'invention consiste alors en un procédé de préparation de mélanges d'acides aminés N-acylés permettant d'obvier aux inconvénients mentionnés ci-dessus. Plus particulièrement le procédé de l'invention permet l'obtention de mélanges d'acides aminés N-acylés pouvant comporter les acides aminés correspondants à tous, ou, si on le souhaite, aux acides aminés principaux, constitutifs d'une protéine choisie, de tels mélanges étant substantiellement exempts de condensat d'acides aminés N-acylés dont le degré de condensation est supérieur à 1. De plus, ces mélanges ne présentent pas d'odeurs désagréables et sont très peu colorés.

De tels mélanges d'acides aminés N-acylés constituent un autre objet de l'invention.

Ces acides aminés de ces mélanges peuvent correspondre aux acides aminés constitutifs de protéines de prix de revient élevé et dont il est difficile de disposer à l'échelle industrielle, sans pour autant que le prix de revient du mélange selon l'invention lui-même n'en soit affecté.

Un troisième objet de l'invention consiste en l'utilisation de ces mélanges d'acides aminés N-acylés dans le domaine des cosmétiques, des produits d'hygiène et des détergents, ainsi qu'en tant qu'agents tensioactifs.

La présente invention consiste en un procédé de préparation d'un mélange d'acides aminés N-acylés dont les fonctions carboxyliques sont libres ou salifiées, caractérisé en ce qu'il comporte les étapes suivantes :
a) on sélectionne chacun des acides aminés constitutifs d'une protéine choisie, lesdits acides aminés se présentant sous forme individualisée,
b) on réalise un mélange à partir des acides aminés sélectionnées à l'étape a),
c) on N-acyle les acides aminés du mélange formé à l'étape b) et, le cas échéant,
d) on salifie les fonctions carboxyliques libres des acides aminés N-acylés.

Les étapes b), c) et d) sont habituellement réalisées en milieu aqueux. Au cours de l'étape a) on sélectionne les acides aminés en fonction du mélange final d'acides aminés N-acylés que l'on souhaite obtenir. Il est important dans le cadre de l'invention que les acides aminés sélectionnés, avant qu'ils ne soient mélangés tel qu'à l'étape b), se présentent substantiellement sous forme individualisée, c'est-à-dire sous forme d'un produit libre et non pas à l'état condensé.

Autrement dit, les acides aminés sélectionnés à l'étape a) ne doivent pas se présenter sous forme de condensat d'acides aminés, tels des oligopeptides, des peptides ou des protéines, dont le degré de condensation est supérieur à 1.

Les acides aminés sous forme individualisée sélectionnés sont généralement disponibles dans le commerce. Ils peuvent être préparés de manière classique, par synthèse chimique ou enzymatique, ou par fermentation. Ils peuvent également être obtenus de manière connue par séparation à partir de mélanges d'acides aminés et d'oligopeptides, résultant de l'hydrolyse d'une protéine ou d'un peptide.

Les acides aminés pouvant être utilisés dans le cadre de la présente invention sont essentiellement des acides α aminés répondant habituellement à la formule générale (I) suivante : 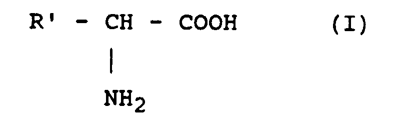 où R' est le reste d'un acide α aminé.

De tels composés sont décrits dans IUPAC Nomenclature of Organic Chemistry, sections A, B, C, D, E and H, 1979 edition, Prepared for publication by J. Rigaudy and S.P. Klesney, pages 193 et 194.

Parmi ces composés, on peut citer plus particulièrement l'alanine, l'arginine, l'acide aspartique, la cystéine, la cystine, la phénylalanine, l'acide glutamique, la glycine, l'histidine, la leucine, l'isoleucine, la lysine, la méthionine, la sérine, la thréonine, le tryptophane, la valine, l'hydroxylysine, la tyrosine, l'asparagine et la glutamine.

D'autres acides α aminés ou leurs dérivés constitutifs des protéines, et ne répondant pas à formule (I) peuvent être utilisées. A ce titre, on peut citer la proline, et l'hydroxyproline. Les acides aminés sélectionnés se présentent généralement sous la forme L ou D,L.

Les acides aminés sélectionnés peuvent plus particulièrement consister en des acides α aminés constitutifs d'une protéine choisie dont la teneur dans ladite protéine est supérieure ou égale à 1 % en poids, ou, supérieure ou égale à 2 % en poids par rapport au poids total des acides aminés présents dans la protéine.

A titre d'exemple, le tableau I ci-après indique des compositions type en acides α aminés (en % en poids) de plusieurs protéines d'origine diverses. Comme indiqué dans ce Tableau I, la protéine de soie est constituée de 17 acides aminés. Selon l'invention, il est possible de sélectionner parmi ces 17 acides aminés, ceux dont la teneur dans cette protéine est supérieure ou égale à 1 % en poids, soit 11 acides aminés, ou seulement ceux dont la teneur est supérieure ou égale à 2 % en poids, soit 7 acides aminés.

Les protéines à partir desquelles on peut déterminer la nature des acides aminés à sélectionner peuvent être d'origine animale, végétale ou être issues d'un micro-organisme. De telles protéines sont indiquées dans le Tableau I. Les protéines dont il est habituellement difficile de disposer de par leur prix et/ou leur rareté, sont avantageusement choisies dans le cadre de l'invention.

De telles protéines sont par exemple celles issues de la soie, de l'olive, du tournesol, d'algues unicellulaires ou multicellulaires, telles les spirulines. Le procédé de l'invention permet alors de reconstituer de telles protéines, à moindre coût.

La manière dont on réalise le mélange à partir des acides aminés sélectionnés n'est pas critique. Habituellement, on introduit chacun des acides aminés dans un solvant, généralement l'eau, contenu dans un récipient, ce sous agitation. L'ordre d'introduction des acides aminés n'est pas critique.

La teneur en poids en chacun des acides aminés peut être quelconque, mais de préférence, elle est voisine, à plus ou moins 50 % près, plus préférentiellement à plus ou moins 10 % près, de la teneur en le même acide aminé dans la protéine choisie.

A titre d'exemple, si l'on sélectionne les acides aminés constitutifs de la protéine de la soie, la teneur en chacun des acides aminés formant le mélange sera à plus ou moins 50 % près, ou à plus ou moins 10 % près la teneur en le même acide aminé lorsqu'il est constitutif de la protéine de la soie. Ainsi, si l'on prend le cas de la glycine, présente en une teneur de 34,65 g pour 100g dans la protéine de la soie, la teneur en cet acide aminé dans le mélange peut être comprise entre 34,65-(0,5 x 34,65) et 34,65 + (0,5 x 34,65) soit entre 17,325 g et 51,975 g pour 100 g d'acides aminés dans le mélange.

En vue de fluidifier le mélange d'acides α aminés ainsi formé, on peut y incorporer un solvant organique comme une cétone telle l'acétone ou un alcool de bas poids moléculaire tel que l'éthanol, ou de préférence l'isopropanol. La teneur en solvant peut être comprise entre 5 et 30 % en poids par rapport à la solution formée. Après quoi on peut réaliser l'acylation des acides α aminés selon tout procédé connu de sorte que chacun des acides α aminés du mélange soit N-acylé par un radical acyle, tel qu'un radical de formule R - CO-dans lequel R représente un reste aliphatique en C₁-C₃₂, de préférence en C₇-C₁₇, saturé ou insaturé, linéaire ou ramifié. Plus particulièrement, R - CO- peut représenter un radical octanoyle, undécylényle, lauroyle, cocoyle, palmitoyle, stéaroyle ou oléoyle.

La réaction d'acylation proprement dite peut être effectuée au moyen d'un agent d'acylation classique après avoir amené le mélange à un pH basique, par exemple un pH compris entre 8 et 12.

L'agent d'acylation peut notamment consister en des dérivés activés d'acides carboxyliques de formule RCOOH, R étant tel que défini ci-dessus. Des dérivés de ce type peuvent consister en l'anhydride symétrique de ces acides ou en des halogénures d'acides comme des chlorures ou des bromures d'acides.

On utilise habituellement de 0,8 à 1 mole d'agent d'acylation par mole d'acides aminés présents dans le milieu réactionnel. La réaction d'acylation peut être effectuée à la pression atmosphérique et à une température comprise entre 0°C et 100°C, de préférence entre 20°C et 60°C. Lors de la réaction d'acylation, le milieu réactionnel est maintenu sous agitation, et avantageusement, on procède de sorte à maintenir son pH basique.

A la fin de la réaction d'acylation, le pH du milieu réactionnel obtenu peut être amené à une valeur inférieure à 3 au moyen d'un acide tel l'acide chlorhydrique, après quoi on peut séparer le mélange d'acides aminés N-acylés du milieu par des méthodes classiques telles la filtration ou la décantation.

Si on le souhaite, on peut salifier les fonctions carboxyliques libres des acides α aminés N-acylés résultant de la réaction d'acylation au moyen d'une base.

Une telle base peut être choisie dans le groupe constitué par :
a) une base organique telle l'arginine, la lysine, la morpholine, la choline, l'ornithine, l'histidine, la mono-, la di- ou la triéthanolamine, et
b) par une base minérale comme l'ammoniaque ou les carbonates, oxydes ou hydroxydes de sodium, potassium, zinc, calcium, aluminium et manganèse ou une solution ammoniacale d'un sel cuivrique.

Un autre objet de la présente invention consiste en un mélange d'acides α aminés N-acylés dont les fonctions carboxyliques sont libres ou salifiées, lesdits acides α aminés N-acylés étant constitués d'acides aminés dont la nature est identique à celle des acides α aminés constitutifs d'une protéine choisie et dont la teneur dans ladite protéine est supérieure ou égale à 0,01 % en poids, ledit mélange étant substantiellement exempt de condensat d'acides α aminés N-acylés.

Lesdits acides α aminés N-acylés peuvent également être tels que la nature des acides α aminés dont ils sont constitués est identique à celle des acides α aminés constitutifs d'une protéine choisie et dont la teneur dans ladite protéine est supérieure ou égale à 1 % en poids, voire supérieure ou égale à 2 % en poids, ces teneurs étant exprimées par rapport au poids total des acides aminés constitutifs de la protéine.

La teneur en poids en un acide α aminé N-acylé donné contenu dans le mélange est avantageusement directement proportionnel à la teneur en poids en le même acide α aminé dans la protéine, ce à 50 % près, de préférence à 10 % près.

De tels mélanges permettent de reconstituer des mélanges d'acides α aminés N-acylés de protéines qu'il était, jusqu'à présent, possible de n'obtenir que sous forme imparfaite, c'est à dire sous forme de mélanges comportant des condensats d'acides α aminés ou de condensats d'acides α aminés N-acylés ou avec certains acides α aminés transformés en leurs dérivés tels qu'indiqués plus haut. Ces mélanges présentent par ailleurs des caractéristiques organoleptiques les rendant aptes à une utilisation en cosmétique ou en hygiène.

Les mélanges d'acides α aminés N-acylés que l'on peut ainsi reconstituer peuvent correspondre à des protéines d'origine animale, végétale ou issues de micro-organismes, telles celles mentionnées ci-dessus.

Le radical acyle constituant les acides α aminés N-acylés du mélange peuvent être ceux de formule R - CO-, R étant tel qu'indiqué ci-dessus. Les fonctions carboxyliques libres des acides α aminés N-acylés peuvent être salifiées par un contre-ion issu d'une base organique ou minérale telles celles citées plus haut.

Lesdits mélanges de l'invention peuvent être préparés selon le procédé décrit ci-dessus.

Selon un autre aspect, l'invention concerne l'utilisation des mélanges d'acides α aminés N-acylés décrits plus haut, pour la préparation de compositions cosmétiques, hygiéniques ou détergentes, telles des crèmes, des laits, des mousses, des aérosols, des gels, des sticks, des huiles, des émulsions, des savons, des lotions aqueuses ou hydroalcooliques, des shampooings, des dentifrices, des compositions détergentes pour la vaisselle ou le linge.

Ces mélanges peuvent être également utilisés en tant qu'agent tensio-actif, notamment quand les fonctions carboxyliques des acides aminés N-acylés sont salifiées par une base minérale comme la soude ou la potasse ou une base organique comme la triéthanolamine.

L'exemple qui suit a pour but d'illustrer la présente invention.

Exemple : mélange reconstitué des acides aminés principaux de la soie, N-acylés par un radical palmitoyle.

On prépare une solution aqueuse d'acides α aminés N-acylés présents dans la protéine de la soie en une teneur supérieure ou égale à 2 % en poids, en introduisant successivement les acides aminés ci-dessous :
- glycine 12,76 g
- alanine 10,69 g
- sérine 5,99 g
- acide aspartique 2,80 g
- acide glutamique 2,35 g
- thréonine 0,95 g
- valine 0,94 g
dans 364 cm³ d'eau dont le pH a été porté à 10,5 au moyen de 46,5 g de soude à 30.% en poids.

Au mélange ainsi obtenu, on ajoute 30 g d'alcool isopropylique, puis simultanément et sous agitation, 104,5 g de chlorure de palmitoyle et 55,3 g de soude à 30 % en poids, à une température comprise entre 40 et 50°C.

Le milieu réactionnel est ensuite maintenu une heure à 60°C sous agitation. On ajoute alors 52,7 g d'acide chlorhydrique à 37 % en poids et on laisse le milieu réactionnel refroidir jusqu'à la température ambiante, sous agitation. La suspension formée est ensuite filtrée, le gâteau lavé jusqu'à ce que les eaux de lavage ne soient plus acides ; puis on sèche sous vide (50 mm de mercure) à 40°C.

On obtient ainsi un mélange des acides aminés principaux de la soie, N-palmitoylé sous forme d'un solide pulvérulent presque blanc et dont l'indice d'acide est de 160.

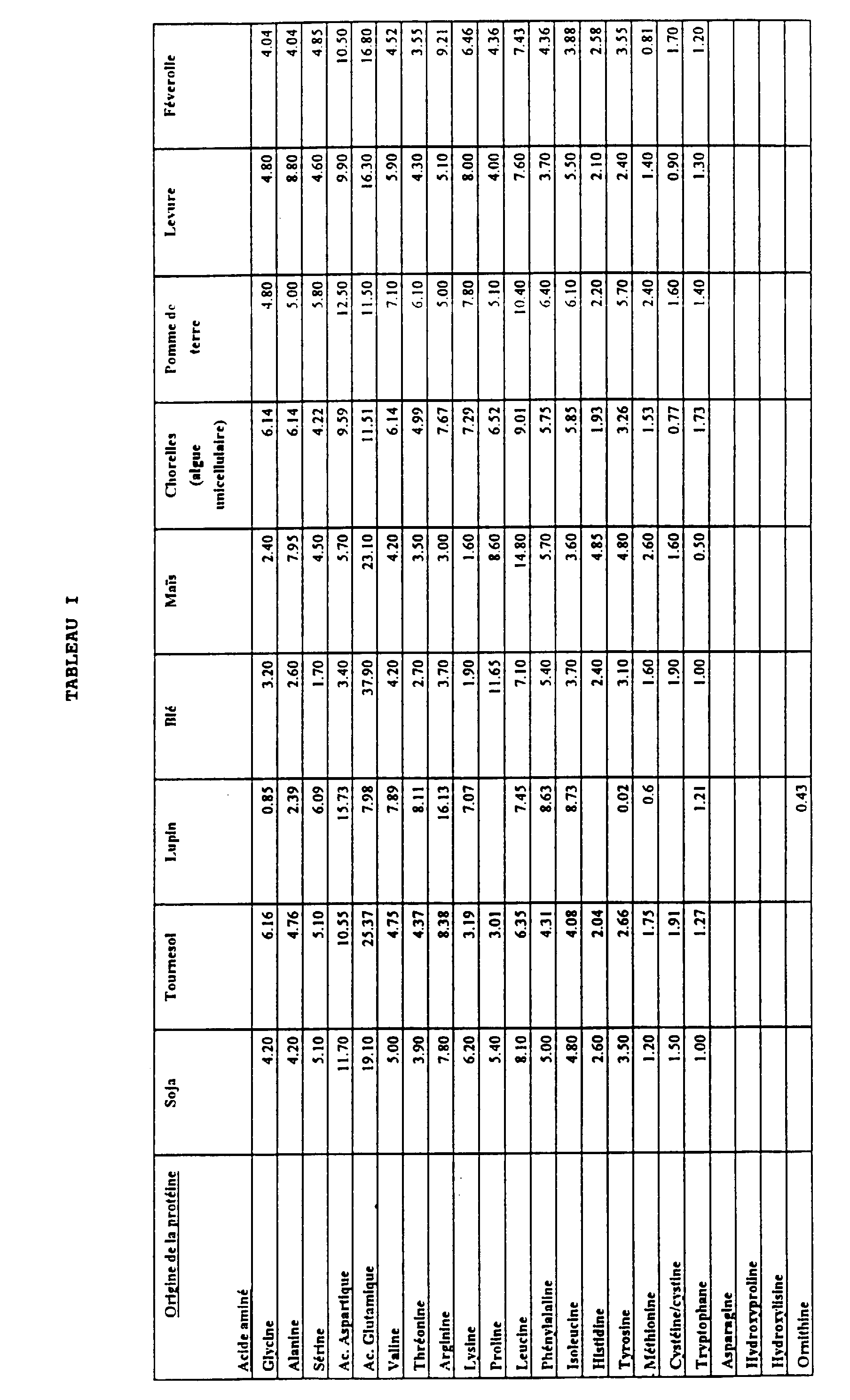

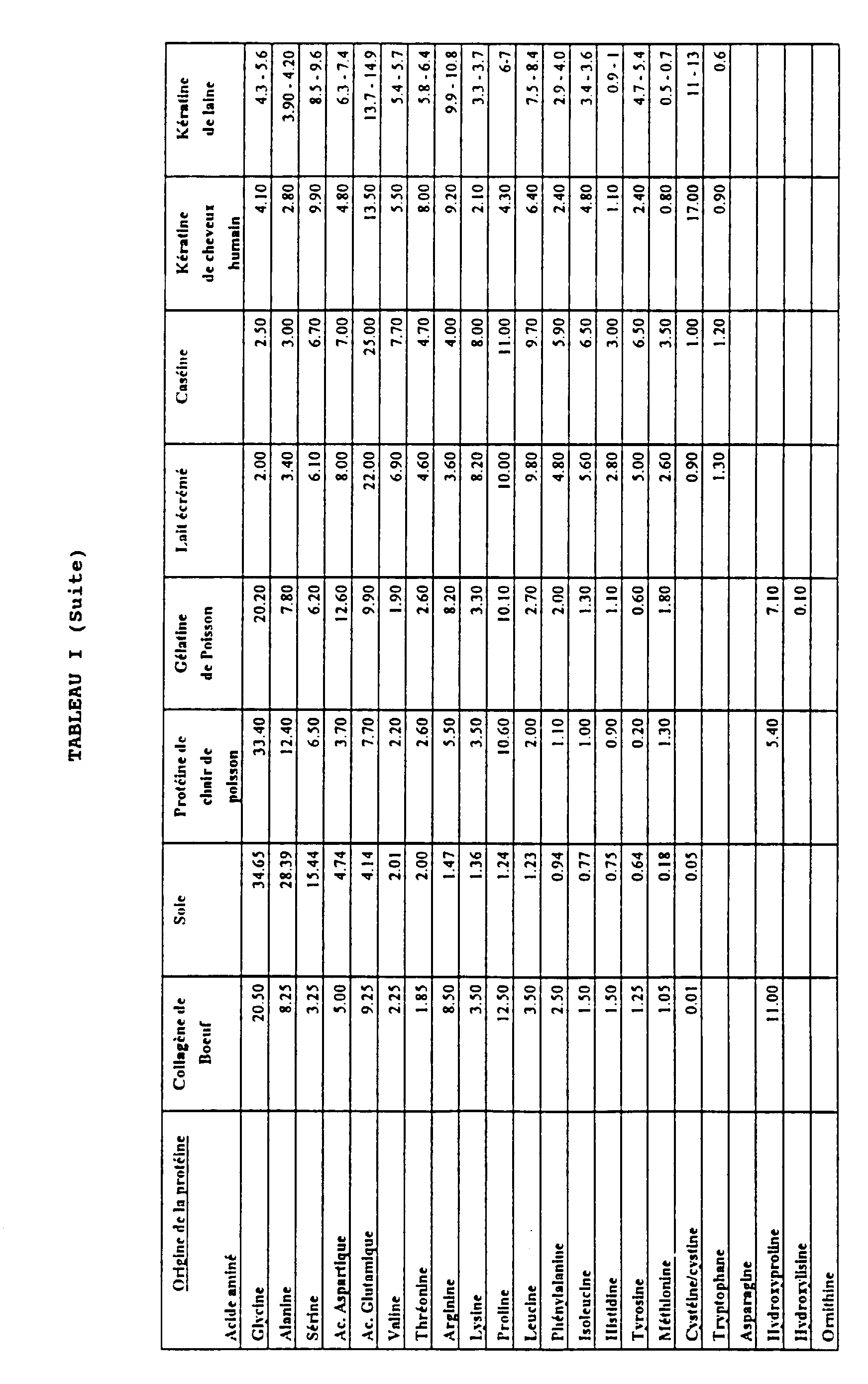

## Revendications

1. Procédé de préparation d'un mélange d'acides aminés N-acylés substantiellement exempts de condensats d'acides aminés N-acylés dont le degré de condensation est supérieur à 1, dont les fonctions carboxyliques sont libres ou salifiées caractérisé en ce qu'il comporte les étapes suivantes :
a) on sélectionne chacun des acides aminés constitutifs d'une protéine choisie, lesdits acides aminés se présentant sous forme individualisée,
b) on réalise dans l'eau un mélange à partir des acides aminés sélectionnés à l'étape a), à pH basique, notamment à un pH compris entre 8 et 12,
c) on N-acyle les acides aminés du mélange formé à l'étape b),
et, le cas échéant,
d) on salifie les fonctions carboxyliques libres des acides aminés N-acylés.

2. Procédé tel que défini à la revendication 1, caractérisé en ce que l'on ajoute un solvant organique à la solution formée à l'étape b), avant de mettre en oeuvre l'étape c).

3. Procédé tel que défini à la revendication 2 dans lequel le solvant organique est une cétone telle que l'acétone ou un alcool de bas poids moléculaire tel que l'ethanol ou l'isopropanol.

4. Procédé selon l'une des revendications 1 à 3, caractérisé en ce que l'on sélectionne chacun des acides aminés constitutifs d'une protéine choisie et dont la teneur dans la protéine est supérieure ou égale à 1 % en poids par rapport au poids total des acides aminés présents dans la protéine.

5. Procédé selon la revendication 4, caractérisé en ce que l'on sélectionne chacun des acides aminés constitutifs d'une protéine choisie et dont la teneur dans la protéine est supérieure ou égale à 2 % en poids par rapport au poids total des acides aminés présents dans la protéine.

6. Procédé selon l'une des revendications 1 à 5, caractérisé en ce que la teneur en un acide aminé donné, contenu dans le mélange formé dans l'étape b), correspond à sa teneur en poids dans la protéine choisie à plus ou moins 50 % près, de préférence à plus ou moins 10 % près.

7. Procédé selon l'une des revendications 1 à 6, caractérisé en ce que la protéine choisie est une protéine d'origine animale, végétale ou issue d'un micro-organisme.

8. Procédé selon la revendication 7, caractérisé en ce que la protéine choisie est une protéine de la soie, de l'olive, du tournesol ou d'une algue.

9. Procédé selon l'une des revendications 1 à 8, caractérisé en ce qu'à l'étape c), chacun des acides aminés du mélange est N-acylé par un radical R - CO-, dans lequel R représente un reste aliphatique, saturé ou insaturé, linéaire ou ramifié, comportant de 1 à 32 atomes de carbone et de préférence, de 7 à 17 atomes de carbone.

10. Procédé selon l'une des revendications 1 à 9, caractérisé en ce que les fonctions carboxyliques des acides aminés sont salifiées par une base choisie dans le groupe constitué par :
a) une base organique telle l'arginine, la lysine, la morpholine, la choline, l'ornithine, l'histidine, la mono-, la di- ou la triéthanolamine, et
b) une base minérale comme l'ammoniaque, les oxydes, carbonates et hydroxydes de sodium, potassium, zinc, calcium, aluminium et manganèse ou une solution ammoniacale d'un sel cuivrique.

11. Un mélange d'acide α-aminés N-acylés dont les fonctions carboxyliques sont libres ou salifiées caractérisé en ce que lesdits acides α-aminés N-acylés sont de nature identique à celle des acides α-aminés constitutifs d'une protéine choisie et dont la teneur dans ladite protéine est supérieure ou égale à 0,01 % en poids, et en ce que ledit mélange est obtenu selon le procédé tel que défini à l'une quelconque des revendications 1 à 10.

12. Un mélange selon la revendication 11, caractérisé en ce que la teneur en chacun des acides α-aminés dans la protéine est supérieure ou égale à 1 % en poids.

13. Un mélange selon l'une des revendications 11 et 12, caractérisé en ce que la teneur de chacun des acides α-aminés dans la protéine est supérieure ou égale à 2 % en poids.

14. Un mélange selon l'une des revendications 11 à 13, caractérisé en ce que la teneur en un acide α-aminé N-acylé donné contenu dans le mélange, est directement proportionnelle à la teneur en poids en le même acide α-aminé dans la protéine, ce à 50 % près, de préférence à 10 % près.

15. Un mélange selon l'une des revendications 11 à 14, caractérisé en ce que la protéine choisie est animale, végétale ou issue d'un micro-organisme.

16. Un mélange selon la revendication 15, caractérisé en ce que la protéine est une protéine de la soie, de l'olive, du tournesol ou d'une algue.

17. Un mélange selon l'une des revendications 11 à 16, caractérisé en ce que les acides α-aminés N-acylés comportent un radical R - CO- dans lequel R représente un reste aliphatique, saturé ou insaturé, linéaire ou ramifié, comportant de 1 à 32 atomes de carbone et de préférence ou 7 à 17 atomes de carbone.

18. Utilisation d'un mélange selon l'une des revendications 11 à 17 pour préparer une composition cosmétique, hygiénique ou détergente.

19. Utilisation d'un mélange selon l'une des revendications 11 à 17 en tant qu'agent tensio-actif.

## Patentansprüche

1. Verfahren zur Herstellung eines N-Acyl-Aminosäuregemisches, das im wesentlichen frei von N-Acyl-Aminosäurekondensaten mit einem Kondensationsgrad größer 1 ist und dessen Carboxylgruppen frei sind oder einer Salzbildung unterworfen wurden, **dadurch gekennzeichnet**, daß es folgende Schritte umfaßt:
a) jede der wesentlichen Aminosäuren eines ausgewählten Proteins wird selektiert, wobei die Aminosäuren individualisiert vorliegen,
b) aus den in Schritt a) selektierten Aminosäuren wird in Wasser ein Gemisch mit basischem pH, insbesondere einem pH zwischen 8 und 12, hergestellt,
c) die Aminosäuren des in Schritt b) hergestellten Gemisches werden N-acyliert, und
d) die freien Carboxylgruppen der N-Acyl-Aminosäuren werden gegebenenfalls einer Salzbildung unterzogen.

2. Verfahren nach Anspruch 1, dadurch gekennzeichnet, daß der in Schritt b) gebildeten Lösung vor Durchführung des Schritts c) ein organisches Lösungsmittel zugegeben wird.

3. Verfahren nach Anspruch 2, bei dem das organische Lösungsmittel ein Keton wie Azeton oder ein Alkohol mit niedrigem Molekulargewicht wie Ethanol oder Isopropanol ist.

4. Verfahren nach einem der Ansprüche 1 bis 3, dadurch gekennzeichnet, daß jede der wesentlichen Aminosäuren eines ausgewählten Proteins selektiert wird, deren Anteil in dem Protein größer oder gleich 1 Gew.-%, bezogen auf das Gesamtgewicht der in dem Protein vorhandenen Aminosäuren, ist.

5. Verfahren nach Anspruch 4, dadurch gekennzeichnet, daß jede der wesentlichen Aminosäuren eines ausgewählten Proteins selektiert wird, deren Anteil in dem Protein größer oder gleich 2 Gew.-%, bezogen auf das Gesamtgewicht der in dem Protein vorhandenen Aminosäuren, ist.

6. Verfahren nach einem der Ansprüche 1 bis 5, dadurch gekennzeichnet, daß der Gehalt an einer gegebenen, in dem in Schritt b) gebildeten Gemisch enthaltenen Aminosäure ihrem Gewichtsanteil ±50%, vorzugsweise ±10%, in dem ausgewählten Protein entspricht.

7. Verfahren nach einem der Ansprüche 1 bis 6, dadurch gekennzeichnet, daß das ausgewählte Protein ein Protein ist, das tierischen oder pflanzlichen Ursprungs ist oder aus einem Mikroorganismus stammt.

8. Verfahren nach Anspruch 7, dadurch gekennzeichnet, daß das ausgewählte Protein ein Seiden-, Oliven-, Lackmus-oder Algenprotein ist.

9. Verfahren nach einem der Ansprüche 1 bis 8, dadurch gekennzeichnet, daß im Schritt c) jede der Aminosäuren des Gemisches mittels eines R-CO-Radikals N-acyliert wird, wobei R einen gesättigten oder ungesättigten, linearen oder verzweigten, aliphatischen Rest darstellt, der 1 bis 32 Kohlenstoffatome und vorzugsweise 7 bis 17 Kohlenstoffatome aufweist.

10. Verfahren nach einem der Ansprüche 1 bis 9, dadurch gekennzeichnet, daß die Carboxylgruppen der Aminosäuren mittels einer Base einer Salzbildung unterzogen werden, welche Base aus der Gruppe ausgewählt wird, die besteht aus:
a) einer organischen Base wie Argenin, Lysin, Morpholin, Cholin, Ornithin, Histidin, Mono-, Di- oder Triethanolamin, und
b) einer anorganische Base wie Ammoniak, Natrium-, Kalium-, Zink-, Calcium-, Aluminium- und Manganoxide, Carbonate und Hydroxide oder eine ammoniakhaltige Lösung eines Kupfersalzes.

11. N-Acyl-α-Aminosäuregemisch, dessen Carboxylgruppen frei sind oder einer Salzbildung unterzogen wurden, dadurch gekennzeichnet, daß die N-Acyl-α-Aminosäuren gleichen Ursprungs sind wie die wesentlichen Aminosäuren eines ausgewählten Proteins und deren Gehalt in dem Protein größer oder gleich 0,01 Gew.-% ist, und daß das Gemisch nach dem Verfahren nach einem der Ansprüche 1 bis 10 gewonnen wird.

12. Gemisch nach Anspruch 11, dadurch gekennzeichnet, daß der Gehalt an jeder α-Aminosäure in dem Protein größer oder gleich 1 Gew.-% ist.

13. Gemisch nach Anspruch 11 oder 12, dadurch gekennzeichnet, daß der Gehalt an jeder der α-Aminosäuren in dem Protein größer oder gleich 2 Gew.-% ist.

14. Gemisch nach einem der Ansprüche 11 bis 13, dadurch gekennzeichnet, daß der Gehalt an einem gegebenen, in dem Gemisch enthaltenen N-Acyl-α-Aminosäure direkt proportional zu dem Gewichtsanteil ±50%, vorzugsweise ±10%, der selben α-Aminosäure in dem Protein ist.

15. Gemisch nach einem der Ansprüche 11 bis 14, dadurch gekennzeichnet, daß das ausgewählte Protein tierisch oder pflanzlich ist oder aus einem Mikroorganismus stammt.

16. Gemisch nach Anspruch 15, dadurch gekennzeichnet, daß das Protein ein Seiden-, Oliven-, Lackmus- oder Algenprotein ist.

17. Gemisch nach einem der Ansprüche 11 bis 16, dadurch gekennzeichnet, daß die N-Acyl-α-Aminosäuren ein R-CO-Radikal umfassen, wobei R einen linearen oder verzweigten, gesättigten oder ungesättigten, aliphatischen Rest darstellt, der 1 bis 32 Kohlenstoffatome und vorzugsweise 7 bis 17 Kohlenstoffatome aufweist.

18. Verwendung eines Gemisches nach einem der Ansprüche 11 bis 17 zur Herstellung einer kosmetischen, hygienischen oder reinigenden Verbindung.

19. Verwendung eines Gemisches nach einem der Ansprüche 11 bis 17 als grenzflächenaktiver Stoff.

## Claims

1. Method for preparing a mixture of N-acylated amino acids substantially free from N-acylated amino acid condensates of which the degree of condensation is greater than 1, of which the carboxyl functional groups are free or converted into salts, characterized in that it comprises the following steps:
a) each of the amino acids constituting a chosen protein is selected, the said amino acids being in an individualized form,
b) a mixture is prepared in water from the amino acids selected in step a), at a basic pH, in particular at a pH of between 8 and 12,
c) the amino acids of the mixture formed in step b) are N-acylated and, optionally,
d) the free carboxyl functional groups of the N-acylated amino acids are converted into salts.

2. Method such as defined in claim 1, characterized in that an organic solvent is added to the solution formed in step b), before putting step c) into operation.

3. Method such as defined in claim 2, wherein the organic solvent is a ketone such as acetone or a lower molecular weight alcohol such as ethanol or isopropanol.

4. Method according to one of claims 1 to 3, characterized in that each of the amino acids constituting a chosen protein is selected of which the concentration in the protein is greater than or equal to 1 % by weight with respect to the total weight of amino acids present in the protein.

5. Method according to claim 4, characterized in that each of the amino acids constituting a chosen protein is selected of which the concentration in the protein is greater than or equal to 2 % by weight with respect to the total weight of amino acids present in the protein.

6. Method according to one of claims 1 to 5, characterized in that the concentration of a given amino acid contained in the mixture formed in step b) corresponds to its concentration by weight in the chosen protein to within plus or minus 50 %, preferably to within plus or minus 10 %.

7. Method according to one of claims 1 to 6, characterized in that the chosen protein is a protein of animal or vegetable origin or is derived from a micro-organism.

8. Method according to claim 7, characterized in that the chosen protein is a protein from silk, olive, sunflower or an alga.

9. Method according to one of claims 1 to 8, characterized in that in step c), each of the amino acids of the mixture is N-acylated by an R - CO- radical, in which R represents a linear or branched, saturated or unsaturated, aliphatic residue, having 1 to 32 carbon atoms and preferably 7 to 17 carbon atoms.

10. Method according to one of claims 1 to 9, characterized in that the carboxyl functional groups of the amino acids are converted into salts by a base chosen from the group consisting of :
a) an organic base such as arginine, lysine, morpholine, choline, ornithine, histidine, mono-, di- or triethanolamine, and
b) an inorganic base such as ammonia, the oxides, carbonates and hydroxides of sodium, potassium, zinc, calcium, aluminium and manganese or an ammoniacal solution of a copper salt.

11. A mixture of N-acylated α-amino acids of which the carboxyl functional groups are free or converted into salts, characterized in that the said N-acylated α-amino acids are of an identical nature to that of the α-amino acids constituting a chosen protein and of which the concentration in the said protein is greater than or equal to 0.01 % by weight, and in that the said mixture is obtained by the process such as defined in any one of claims 1 to 10.

12. A mixture according to claim 11, characterized in that the concentration of each of the α-amino acids in the protein is greater than or equal to 1 % by weight.

13. A mixture according to either of claims 11 or 12, characterized in that the concentration of each of the α-amino acids in the protein is greater than or equal to 2 % by weight.

14. A mixture according to one of claims 11 to 13, characterized in that the concentration of a given N-acylated α-amino acid contained in the mixture is directly proportional to the concentration by weight of the same α-amino acid in the protein, to within 50 %, preferably to within 10 %.

15. A mixture according to one of claims 11 to 14, characterized in that the chosen protein is animal, vegetable or derived from a micro-organism.

16. A mixture according to claim 15, characterized in that the protein is a protein from silk, olive, sunflower or an alga.

17. A mixture according to one of claims 11 to 16, characterized in that the N-acylated α-amino acids include an R-CO-radical in which R represents a linear or branched, saturated or unsaturated, aliphatic residue, having 1 to 32 carbon atoms and preferably 7 to 17 carbon atoms.

18. Use of a mixture according to one of claims 11 to 17 for the preparing a cosmetic, hygienic or detergent composition.

19. Use of a mixture according to one of claims 11 to 17 as a surface active agent.
